# EUROPEAN PATENT SPECIFICATION

(11) **EP 1 348 702 B1**
(45) Date of publication and mention of the grant of the patent: **20.12.2006**
(21) Application number: 03006822.5
(22) Date of filing: 27.03.2003
(51) Int. Cl.: C07D 285/24

(54) **Method of producing 1, 2, 4-benzothiadiazine-1, 1-dioxide compound**
Verfahren zur Herstellung von 1,2,4-Benzothiadiazin-1,1-Dioxid Derivate
Procédé poUr la préparation de dérivés de la 1,2,4-Benzothiadiazin-1,1-Dioxide

(30) Priority: 29.03.2002 JP 2002095641
(43) Date of publication of application: 01.10.2003
(73) Proprietor: FUJI PHOTO FILM CO., LTD., Minami-Ashigara-shi, Kanagawa-ken (JP)
(72) Inventor: Kaneko, Yushi, Fuji Photo Film Co., Ltd., Kanagawa-ken (JP); Motoki, Masuji, Fuji Photo Film Co., Ltd., Kanagawa-ken (JP); Yamakawa, Katsuyoshi, Fuji Photo Film Co., Ltd., Kanagawa-ken (JP); Mori, Hideto, Fuji Photo Film Co., Ltd., Kanagawa-ken (JP); Yasuoka, Hiroshi, Fuji Photo Film Co., Ltd., Kanagawa-ken (JP)
(74) Representative: HOFFMANN EITLE

(56) References cited:
- US-A- 3 841 880
- KUMAR, C.V. ET AL.: "Photoinduced Ring Enlargement Reactions of 2H-Benzothiadiazine 1,1-Dioxides. Steady-State and Laser Flash Photolysis Studies" J. ORG. CHEM., vol. 51, 1986, pages 1967-1972, XP002240312
- TOPLISS, S. ET AL.: "Antihypertensive Agents. I. Non-diuretic 2H-1,2,4-Benzothiadiazine 1,1-Dioxides" J. MED. CHEM., vol. 6, 1963, pages 122-127, XP002240313
- TOPLISS J G ET AL: "3-Substituted Dihydrobenzothiadiazine 1,1-Dioxides as Diuretic Agents" JOURNAL OF ORGANIC CHEMISTRY, AMERICAN CHEMICAL SOCIETY. EASTON, US, vol. 26, 1961, pages 3842-3850, XP002107971 ISSN: 0022-3263

## Description

### FIELD OF THE INVENTION

The present invention relates to a method of producing 1,2,4-benzothiadiazine-1,1-dioxide compounds useful as a yellow dye-forming coupler that can be used in a silver halide photosensitive light-sensitive material, and useful as a synthesis intermediate thereof, and also useful as synthesis intermediates of other compounds, such as dyes, pharmaceuticals, agricultural chemicals, electronic materials, and the like.

### BACKGROUND OF THE INVENTION

As described in U.S. Patent No. 3,841,880, 1,2,4-benzothiadiazine-1,1-dioxide compounds containing a substituted or unsubstituted alkyl or aryl group in the 2-position and a substituted or unsubstituted alkyl group in the 3-position, are useful as a yellow dye-forming coupler that can be used in a silver halide photosensitive light-sensitive material or as a synthesis intermediate of the said coupler, and also useful as synthesis intermediates of other compounds, such as dyes, pharmaceuticals, agricultural chemicals, electronic materials, and the like.

As a known synthesis method of such compounds, the aforementioned U.S. Patent No. 3,841,880 discloses a method of producing 3-ethoxycarbonylmethyl-1,2,4-benzothiadiazine-1,1-dioxide by a reaction between 2-aminobenzene sulfonamide and ethyl β,β-diethoxyacrylate. However, in a method of synthesizing 1,2,4-benzothiadiazine-1,1-dioxide compounds having a substituent in the 2-position, there is a problem in that both a high reaction temperature and a long reaction time are needed to complete a ring closure reaction. Further, the aforementioned U.S. Patent discloses that a reaction is accelerated by addition of acetic acid. However, the effect of acetic acid is not satisfactory.

Further, a method of subjecting a 1,2,4-thiadiazine-1,1-dioxide compound, containing a hydrogen atom in the 2-position (or 4-position), to alkylation, is known, as described in J. Chin. Chem. Soc., 45, 805 (1998). However, there is also a problem in that this method produces a mixture of a compound having an alkyl group in the 2-position and a compound having an alkyl group in the 4-position. Further, methods of utilizing a reaction of a 2-aminobenzene sulfonamide compound with an orthoester compound are known, as described in J. Med. Chem., 6, 122 (1963), J. Org. Chem., 51, 1967 (1986), and Tetrahedron, 39, 2073 (1983). However, each of these methods also have a problem that a high reaction temperature is needed to complete the reaction. Further, J. Org. Chem., 16, 815 (1951) also describes a method using a reaction of a 2-aminobenzene sulfonamide compound with an orthoester compound. However, there is also a problem that this reaction is low in yield. Further, Huaxue Shiji, 21, 235 (1999) describes a method of subjecting a 2-aminobenzenesulfonamide compound to acylation with an acylating agent, followed by cyclization with phosphorus oxychloride. However, this method has also a problem that the synthesis must be conducted via many steps, and that when the acyl portion contains a functional group, the yield attained in cyclization step is low.

### SUMMARY OF THE INVENTION

Accordingly, an object of the present invention is to provide a method of producing 1,2,4-benzothiadiazine-1,1-dioxide compounds from readily obtainable raw materials, in short steps, under mild reaction conditions, with good yield.

Other and further features and advantages of the invention will appear more fully from the following description.

### DETAILED DESCRIPTION OF THE INVENTION

As a result of intensive studies to solve the aforementioned problems, the present inventors have found that 1,2,4-benzothiadiazine-1,1-dioxide compounds represented by formula (I) shown below can be synthesized according to the following steps. First, a 2-aminobenzenesulfonamide compound represented by formula (III) as shown below is prepared from inexpensive raw materials. Through use of the thus-prepared compound as a reactant, an iminoether compound represented by formula (II) shown below is obtained as an intermediate. Then, the intermediate is subjected to a ring closure reaction, in the presence of a base, to obtain the aforementioned objective compound. The present invention has been completed based on this finding.

According to the present invention, there is provided the following means:
(1) A method of producing a 1,2,4-benzothiadiazine-1,1-dioxide compound represented by formula (I), comprising reacting a 2-aminobenzenesulfonamide compound represented by formula (III) with a 1,1-dialkoxyalkene compound represented by formula (IV) or an orthoester compound represented by formula (V), in the presence of an acid, to obtain an iminoether compound represented by formula (II), and then subjecting the iminoether compound to a ring closure reaction, in the presence of a base: 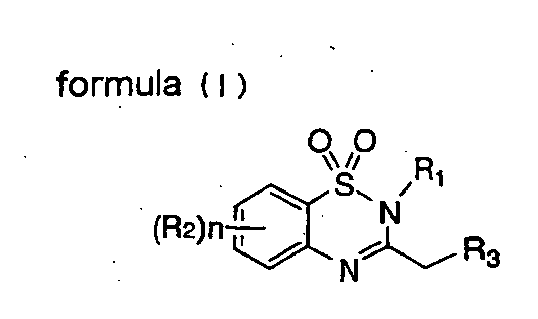 wherein, in formula (I), R₁ represents a substituted' or unsubstituted alkyl group, or a substituted or unsubstituted aryl group; R₂ represents a substituent; n represents an integer of 0 to 4; when n is 2 or more, R₂s may be the same or different, and R₂s may bond with each other to form a ring; and R₃ represents a hydrogen atom or a substituent; 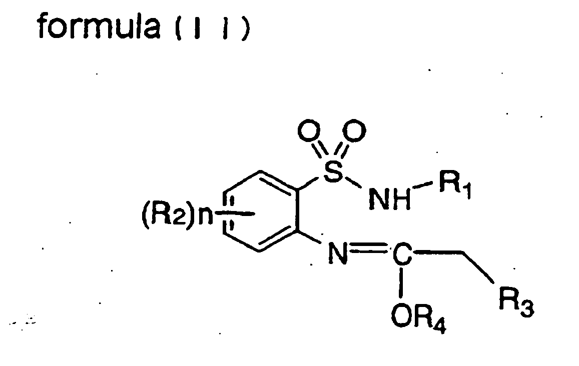 wherein, in formula (II), R₁ represents a substituted or unsubstituted alkyl group, or a substituted or unsubstituted aryl group; R₂ represents a substituent; n represents an integer of 0 to 4; when n is 2 or more, R₂s may be the same or different, and R₂s may bond with each other to form a ring; R₃ represents a hydrogen atom or a substituent; and R₄ represents a substituted or unsubstituted alkyl group; 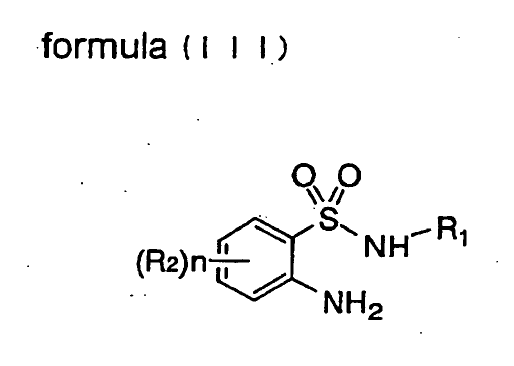 wherein, in formula (III), R₁ represents a substituted or unsubstituted alkyl group, or a substituted or unsubstituted aryl group; R₂ represents a substituent; n represents an integer of 0 to 4; and when n is 2 or more, R₂s may be the same or different, and R₂s may bond with each other to form a ring; 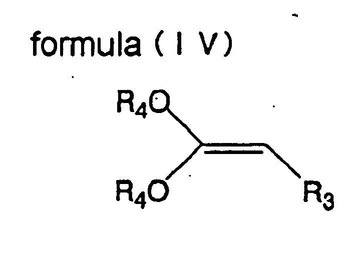 wherein, in formula (IV), R₃ represents a hydrogen atom or a substituent; and R₄ represents a substituted or unsubstituted alkyl group; and 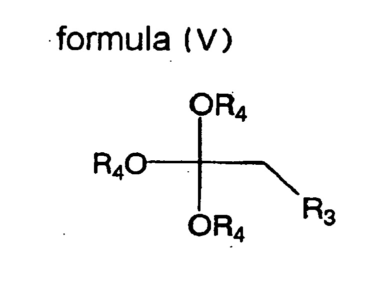 wherein, in formula (V), R₃ represents a hydrogen atom or a substituent; and R₄ represents a substituted or unsubstituted alkyl group.
(2) A method of producing a 1,2,4-benzothiadiazine-1,1-dioxide compound represented by formula (I), comprising subjecting an iminoether compound represented by formula (II) to a ring closure reaction, in the presence of a base: 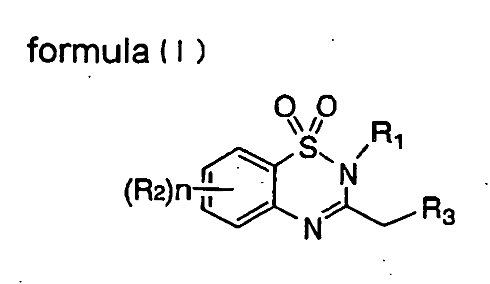
wherein, in formula (I), R₁ represents a substituted or unsubstituted alkyl group, or a substituted or unsubstituted aryl group; R₂ represents a substituent; n represents an integer of 0 to 4; when n is 2 or more, R₂s may be the same or different, and R₂s may bond with each other to form a ring; and R₃ represents a hydrogen atom or a substituent; 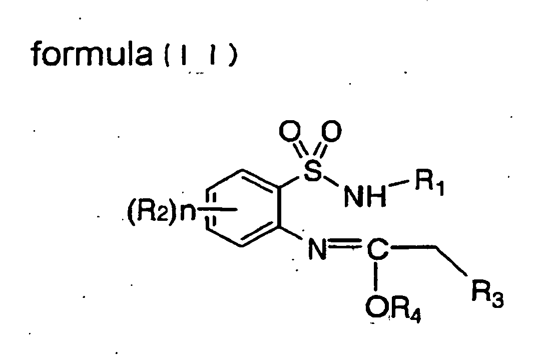 wherein, in formula (II), R₁ represents a substituted or unsubstituted alkyl group, or a substituted or unsubstituted aryl group; R₂ represents a substituent; n represents an integer of 0 to 4; when n is 2 or more, R₂s may be the same or different, and R₂s may bond with each other to form a ring; R₃ represents a hydrogen atom or a substituent; and R₄ represents a substituted or unsubstituted alkyl group.

The present invention is explained in detail below.

The compound represented by formula (I) according to the present invention is explained in detail.

In formula (I), R₂ represents a substituent. n represents an integer of 0 to 4. When n is 2 or more, R₂s may be the same or different, and R₂s may bond with each other to form a ring. Examples of the substituent represented by R₂ include a halogen atom, an alkyl group (including a cycloalkyl group and a bicycloalkyl group), an alkenyl group (including a cycloalkenyl group and a bicycloalkenyl group), an alkynyl group, an aryl group, a heterocyclic group, a cyano group, a hydroxyl group, a nitro group, a carboxyl group, an alkoxy group, an aryloxy group, a silyloxy group, a heterocyclic oxy group, an acyloxy group, a carbamoyloxy group, an alkoxycarbonyloxy group, an aryloxycarbonyloxy group, an amino group (including an alkylamino group and an anilino group), an acylamino group, an aminocarbonylamino group, an alkoxycarbonylamino group, an aryloxycarbonylamino group, a sulfamoylamino group, an alkyl- or aryl-sulfonylamino group, a mercapto group, an alkylthio group, an arylthio group, a heterocyclic thio group, a sulfamoyl group, a sulfo group, an alkyl- or aryl-sulfinyl group, an alkyl- or aryl-sulfonyl group, an acyl group, an aryloxycarbonyl group, an alkoxycarbonyl group, a carbamoyl group, an arylazo group or a heterocyclicazo group, an imido group, a phosphino group, a phosphinyl group, a phosphinyloxy group, a phosphinylamino group, and a silyl group.

The above-mentioned substituent may be further substituted with another substituent. Examples of this another substituent are the same as described as the examples of the above-mentioned substituent.

Examples of the substituent represented by R₂ are further explained below.

Examples of the substituent include a halogen atom (e.g., a chlorine atom, a bromine atom, an iodine atom); an alkyl group (a straight- or branched-chain, substituted or unsubstituted alkyl group, and preferably an alkyl group having 1 to 30 carbon atoms, e.g., methyl, ethyl, n-propyl, isopropyl, t-butyl, n-octyl, eicosyl, 2-chloroethyl, 2-cyanoethyl, 2-ethylhexyl, and 3-(2,4-di-t-amylphenoxy)propyl); a cycloalkyl group (preferably a substituted or unsubstituted monocyclic cycloalkyl group having 3 to 30 carbon atoms, e.g., cyclohexyl, cyclopentyl, 4-n-dodecyl cyclohexyl; and polycyclic cycloalkyl groups including groups having a polycyclic structure, such as a bicycloalkyl group (preferably a substituted or unsubstituted bicycloalkyl group having 5 to 30 carbon atoms, e.g. bicyclo[1,2,2]heptane-2-yl and bicyclo[2,2,2]octane-3-yl), and a tricycloalkyl group; more preferably a monocyclic cycloalkyl group and a bicycloalkyl group, and particularly preferably a monocyclic cycloalkyl group); an alkenyl group (a straight- or branched-chain, substituted or unsubstituted alkenyl group, preferably having 2 to 30 carbon atoms, e.g., vinyl, allyl, prenyl, geranyl, oleyl); a cycloalkenyl group (preferably a substituted or unsubstituted monocyclic cycloalkenyl group having 3 to 30 carbon atoms, e.g., 2-cyclopentene-1-yl, 2-cyclohexene-1-yl; and a polycyclic cycloalkenyl group, such as a bicycloalkenyl group (preferably a substituted or unsubstituted bicycloalkenyl group having 5 to 30 carbon atoms, e.g., bicyclo[2,2,1]hepto-2-ene-1-yl and bicyclo[2,2,2]octo-2-ene-4-yl) and a tricycloalkenyl group, with a monocyclic cycloalkenyl group being particularly preferred); an alkynyl group (preferably a substituted or unsubstituted alkynyl group having 2 to 30 carbon atoms, e.g., ethynyl, propargyl, trimethylsilylethynyl); an aryl group (preferably a substituted or unsubstituted aryl group having 6 to 30 carbon atoms, e.g., phenyl, p-tolyl, naphthyl, m-chlorophenyl, o-hexadecanoylaminophenyl); a heterocyclic group (preferably a 5- to 7-membered, substituted or unsubstituted, saturated or unsaturated, aromatic or non-aromatic, monocyclic or condensed heterocyclic group, more preferably a heterocyclic group having ring-constituting atoms selected from carbon, nitrogen, and sulfur atoms, and containing at least one hetero atom selected from the group consisting of nitrogen, oxygen, and sulfur atoms, further preferably a 5- or 6-membered aromatic heterocyclic group having 3 to 30 carbon atoms; e.g., 2-furyl, 2-thienyl, 2-pyridyl, 4-pyridyl, 2-pyrimidinyl, 2-benzothiazolyl); a cyano group; a hydroxyl group; a nitro group; a carboxyl group; an alkoxy group (preferably a substituted or unsubstituted alkoxy group having 1 to 30 carbon atoms, e.g., methoxy, ethoxy, isopropoxy, t-butoxy, n-octyloxy, 2-methoxyethoxy); an aryloxy group (preferably a substituted or unsubstituted aryloxy group having 6 to 30 carbon atoms, e.g., phenoxy, 2-methylphenoxy, 2,4-di-t-amylphenoxy, 4-t-butylphenoxy, 3-nitrophenoxy, 2-tetradecanoylaminophenoxy); a silyloxy group (preferably a silyloxy group having 3 to 20 carbon atoms, e.g., trimethylsilyloxy, t-butyldimethylsilyloxy); a heterocyclic oxy group (preferably a substituted or unsubstituted heterocyclic oxy group having 2 to 30 carbon atoms, more preferably having the same heterocycle moiety as that of the heterocyclic group, e.g., 1-phenyltetrazole-5-oxy, 2-tetrahydropyranyloxy); an acyloxy group (preferably formyloxy, a substituted or unsubstituted alkylcarbonyloxy group having 2 to 30 carbon atoms, a substituted or unsubstituted arylcarbonyloxy group having 6 to 30 carbon atoms, e.g., formyloxy, acetyloxy, pivaloyloxy, stealoyloxy, benzoyloxy, p-methoxyphenylcarbonyloxy); a carbamoyloxy group (preferably a substituted or unsubstituted carbamoyloxy group having 1 to 30 carbon atoms, e.g., N,N-dimethylcarbamoyloxy, N,N-diethylcarbamoyloxy, morpholino carbonyloxy, N,N-di-n-octylaminocarbonyloxy, N-n-octylcarbamoyloxy); an alkoxycarbonyloxy group (preferably a substituted or unsubstituted alkoxycarbonyloxy group having 2 to 30 carbon atoms, e.g., methoxycarbonyloxy, ethoxycarbonyloxy, t-butoxy carbonyloxy, n-octylcarbonyloxy); an aryloxycarbonyloxy group (preferably a substituted or unsubstituted aryloxycarbonyloxy group having 7 to 30 carbon atoms, e.g., phenoxycarbonyloxy, p-methoxy phenoxycarbonyloxy, p-n-hexadecyloxyphenoxy carbonyloxy); an amino group (preferably an amino group, a substituted or unsubstituted alkylamino group having 1 to 30 carbon atoms, a substituted or unsubstituted arylamino group having 6 to 30 carbon atoms, and a heterocyclic amino group having 0 to 30 carbon atoms, e.g., amino, methylamino, dimethylamino, anilino, N-methyl-anilino, diphenylamino, N-1,3,5-triazine-2-yl amino), an acylamino group (preferably formylamino group, a substituted or unsubstituted alkylcarbonylamino group having 1 to 30 carbon atoms, a substituted or unsubstituted arylcarbonylamino group having 6 to 30 carbon atoms, e.g., formylamino, acetylamino, pivaloylamino, lauroylamino, benzoylamino, and 3,4,5-tri-n-octyloxyphenylcarbonylamino); an aminocarbonylamino group (preferably a substituted or unsubstituted aminocarbonylamino group having 1 to 30 carbon atoms, e.g., carbamoylamino, N,N-dimethylaminocarbonylamino, N,N-diethylamino carbonylamino, morpholinocarbonylamino), an alkoxycarbonylamino group (preferably a substituted or unsubstituted alkoxycarbonylamino group having 2 to 30 carbon atoms, e.g., methoxycarbonylamino, ethoxy carbonylamino, t-butoxycarbonylamino, n-octadecyloxycarbonylamino, N-methyl-methoxycarbonylamino); an aryloxycarbonylamino group (preferably a substituted or unsubstituted aryloxycarbonylamino group having 7 to 30 carbon atoms, e.g., phenoxycarbonylamino, p-chlorophenoxycarbonylamino, m-n-octyloxyphenoxycarbonyl amino); a sulfamoyl amino group (preferably a substituted or unsubstituted sulfamoylamino group having 0 to 30 carbon atoms, e.g., sulfamoylamino, N,N-dimethylaminosulfonylamino, N-n-octyl aminosulfonylamino); an alkyl- or aryl-sulfonylamino group (preferably a substituted or unsubstituted alkyl-sulfonylamino group having 1 to 30 carbon atoms, and a substituted or unsubstituted aryl-sulfonylamino group having 6 to 30 carbon atoms, e.g., methylsulfonylamino, butylsulfonylamino, phenylsulfonylamino, 2,3,5-trichlorophenylsulfonylamino, p-methylphenylsulfonylamino); a mercapto group; an alkylthio group (preferably a substituted or unsubstituted alkylthio group having 1 to 30 carbon atoms, e.g., methylthio, ethylthio, n-hexadecylthio), an arylthio group (preferably a substituted or unsubstituted arylthio group having 6 to 30 carbon atoms, e.g., phenylthio, p-chlorophenylthio, m-methoxyphenylthio); a heterocyclic thio group (preferably a substituted or unsubstituted heterocyclic thio group having 2 to 30 carbon atoms in which the heterocycle moiety is preferably the same as that of the above-described heterocyclic group, e.g., 2-benzothiazolylthio, 1-phenyltetrazol-5-ylthio); a sulfamoyl group (preferably a substituted or unsubstituted sulfamoyl group having 0 to 30 carbon atoms, e.g., N-ethylsulfamoyl, No(3-dodecyloxypropyl)sulfamoyl, N,N-dimethyl sulfamoyl, N-acetylsulfamoyl, N-benzoylsulfamoyl, N-(N'-phenylcarbamoyl)sulfamoyl); a sulfo group; an alkyl- or aryl-sulfinyl group (preferably a substituted or unsubstituted alkylsulfinyl group having 1 to 30 carbon atoms, and a substituted or unsubstituted arylsulfinyl group having 6 to 30 carbon atoms, e.g., methyl sulfinyl, ethyl sulfinyl, phenylsulfinyl, p-methylphenylsulfinyl); an alkyl- or aryl-sulfonyl group (preferably a substituted or unsubstituted alkyl sulfonyl group having 1 to 30 carbon atoms, and a substituted or unsubstituted arylsulfonyl group having 6 to 30 carbon atoms, e.g., methylsulfonyl, ethylsulfonyl, phenylsulfonyl, p-methylphenylsulfonyl); an acyl group (preferably a formyl group, a substituted or unsubstituted alkylcarbonyl group having 2 to 30 carbon atoms, and a substituted or unsubstituted arylcarbonyl group having 7 to 30 carbon atoms, e.g., acetyl, pivaloyl, 2-chloroacetyl, stearoyl, benzoyl, p-n-octyloxyphenylcarbonyl); an aryloxycarbonyl group (preferably a substituted or unsubstituted aryloxycarbonyl group having 7 to 30 carbon atoms, e.g., phenoxycarbonyl, o-chlorophenoxycarbonyl, m-nitrophenoxy carbonyl, p-t-butylphenoxycarbonyl); an alkoxycarbonyl group (preferably a substituted or unsubstituted alkoxycarbonyl group having 2 to 30 carbon atoms, e.g., methoxycarbonyl, ethoxycarbonyl, t-butoxycarbonyl, n-octadecyloxycarbonyl); a carbamoyl group (preferably a substituted or unsubstituted carbamoyl group having 1 to 30 carbon atoms, e.g., carbamoyl, N-methylcarbamoyl, N,N-dimethylcarbamoyl, N,N-di-n-octylcarbamoyl, N-(methylsulfonyl)carbamoyl); an aryl azo group or heterocyclic azo group (preferably a substituted or unsubstituted aryl azo group having 6 to 30 carbon atoms, and a substituted or unsubstituted heterocyclic azo group having 3 to 30 carbon atoms, in which the heterocyclic moiety is preferably the same as that of the above-described heterocyclic group, e.g., phenylazo, p-chlorophenylazo, 5-ethylthio-1,3,4-thiadiazole-2-yl azo); an imido group (preferably a substituted or unsubstituted imido group having 2 to 30 carbon atoms, e.g., N-succinimido, N-phthalimido); a phosphino group (preferably a substituted or unsubstituted phosphino group having 2 to 30 carbon atoms, e.g., dimethylphosphino, diphenylphosphino, methylphenoxyphosphino); a phosphinyl group (preferably a substituted or unsubstituted phosphinyl group having 2 to 30 carbon atoms, e.g., phosphinyl, dioctyloxyphosphinyl, diethoxyphosphinyl); a phosphinyloxy group (preferably a substituted or unsubstituted phosphinyloxy group having 2 to 30 carbon atoms, e.g., diphenoxyphosphinyloxy, dioctyloxyphosphinyloxy); a phosphinylamino group (preferably a substituted or unsubstituted phosphinylamino group having 2 to 30 carbon atoms, e.g., dimethoxyphosphinylamino, dimethylamino phosphinylamino); and a silyl group (preferably a substituted or unsubstituted silyl group having 3 to 30 carbon atoms, e.g., trimethylsilyl, t-butyl dimethylsilyl, phenyldimethylsilyl).

Among the above-described groups, a hydrogen atom(s) possessed in the group may be removed to replace with any one of the above-described groups. Examples of such the group include an alkylcarbonylaminosulfonyl group, an arylcarbonylaminosulfonyl group, an alkylsulfonylaminocarbonyl group, and an arylsulfonyl aminocarbonyl group. As the specific examples, methylsulfonyl aminocarbonyl, p-methylphenylsulfonylaminocarbonyl, acetylaminosulfonyl, and benzoylaminosulfonyl groups are enumerated.

In formula (I), R₁ represents a substituted or unsubstituted alkyl group or a substituted or unsubstituted aryl group. Examples of the substituent that R₁ may have are those atoms and groups exemplified as a substituent represented by R₂.

R₁ ist preferably a substituted or unsubstituted alkyl group. When R₁ is an alkyl group, a total of carbon atoms of R₁ is preferably 1 to 60, more preferably 1 to 50, still more preferably 1 to 40. When R₁ is an aryl group, a total of carbon atoms of R₁ is preferably 6 to 60, more preferably 6 to 50, still more preferably 6 to 40.

In the compound represented by formula (I), R₃ represents a hydrogen atom or a substituent. Examples of the substituent are those atoms and groups exemplified as the substituent represented by R₂. R₃ is preferably a hydrogen atom, an alkyl group, an aryl group, an alkoxycarbonyl group, an aryloxycarbonyl group, and a carbamoyl group, and more preferably a hydrogen atom, an alkyl group, and an alkoxycarbonyl group.

Next, the compound represented by formula (II) according to the present invention is explained in detail.

In formula (II), R₁, R₂, R₃, and n each have the same meanings as described in formula (I). A preferable range of each symbol is the same as in formula (I).

In formula (II), R₄ represents a substituted or unsubstituted alkyl group. R₄ is preferably an unsubstituted alkyl group, more preferably a methyl group or an ethyl group, and most preferably an ethyl group.

Here, the present invention embraces tautomers, in which a hydrogen atom on the carbon atom of the methylene moiety that constitutes a part of the -N=C(OR₄)-CH₂-moiety of the compound represented by formula (II) is transferred onto the nitrogen atom to form a -NH-C(OR₄)=CH- moiety. The chemical formulae shown in the present specification are described taking one of these tautomers for convenience. Accordingly, all possible tautomers are embraced in the present invention.

Next, the compound represented by formula (III) according to the present invention is explained in detail.

In formula (III), R₁, R₂, and n each have the same meanings as described in formula (I). A preferable range of each symbol is the same as in formula (I).

Next, the compounds represented by formula (IV) or (V) according to the present invention are explained in detail.

In formula (IV) or (V), R₃ has the same meaning as described in formula (I), and its preferable range is the same as in formula (I). R₄ has the same meaning as described in formula (II), and its preferable range is the same as in formula (II). In each of the aforementioned chemical formulae, there is a plurality of R₄s. These R₄s may be the same or different from each other. In the present invention, it is preferable that all R₄s in the same molecule are the same.

The compound represented by formula (I) may contain one, or two or more asymmetric carbon atoms according to the kind of substituent. Any kind of optical isomers or diastereoisomers owing to these asymmetric carbon atoms are also embraced in the present invention. Further, in addition to the isomers in pure (unmixed) form, isomers in other forms, such as any mixtures of these pure isomers and racemic modification, are also embraced in the present invention. When the aforementioned compound of the present invention contains one, or two or more double bonds, any geometrical isomers owing to these double bonds are also embraced in the present invention.

Among the compounds represented by formula (I) according to the present invention, preferable specific examples of the compounds are shown below, but the present invention is not limited to these compounds. Note that the present invention embraces tautomers in which a hydrogen atom, of the active methylene moiety (a hydrogen atom on the carbon atom to which R₃ is substituting), is transferred onto the nitrogen atom of the C=N moiety in the 1,2,4-benzothiadiazine-1,1-dione ring (i.e. the nitrogen atom in the 4-position of the said ring). The chemical formulae shown herein are described taking one of these tautomers, for convenience. Accordingly, all possible tautomers are embraced in the present invention.

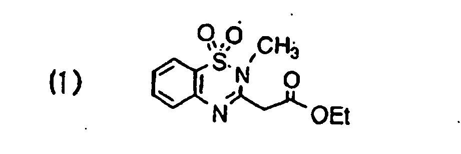

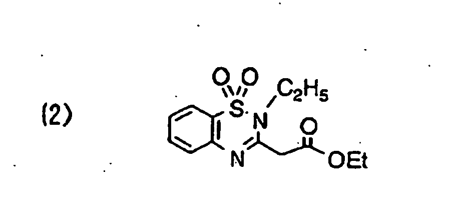

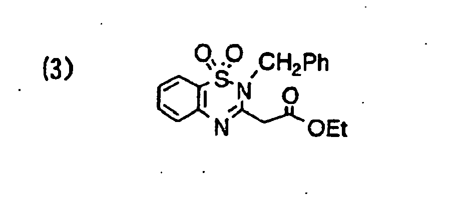

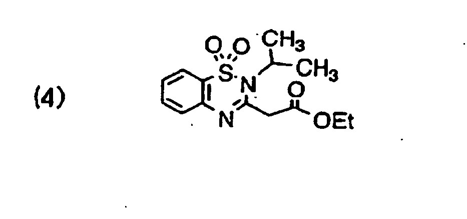

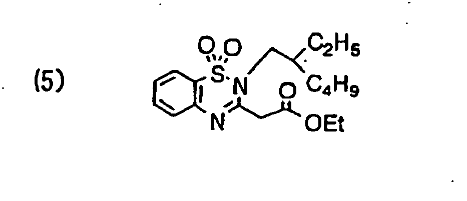

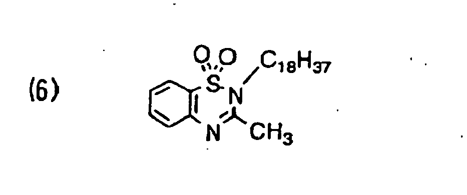

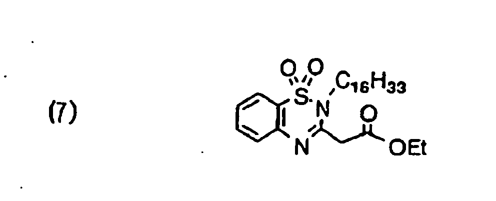

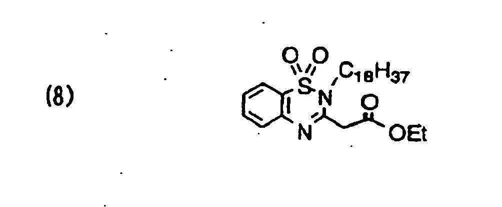

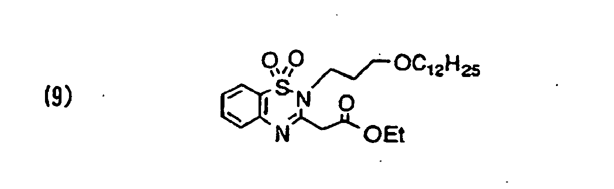

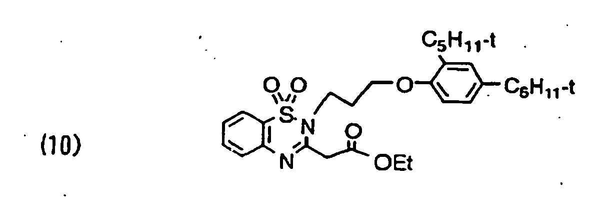

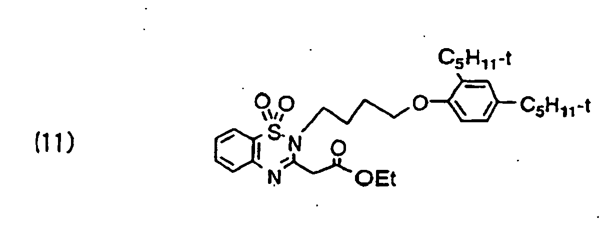

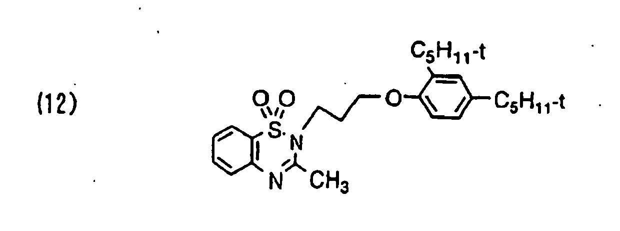

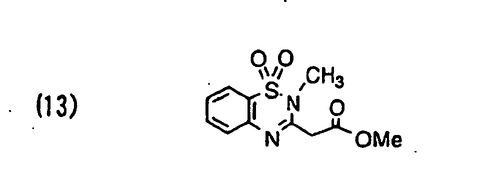

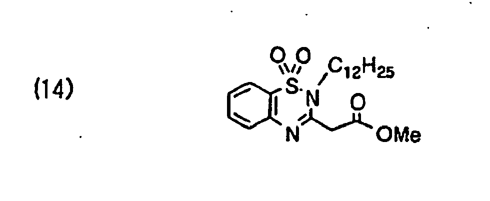

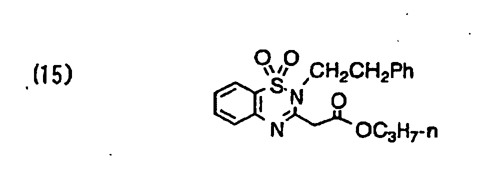

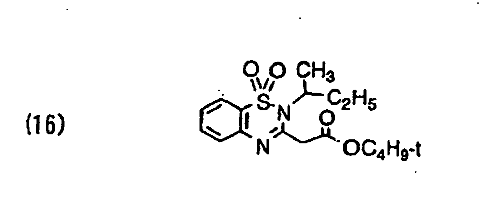

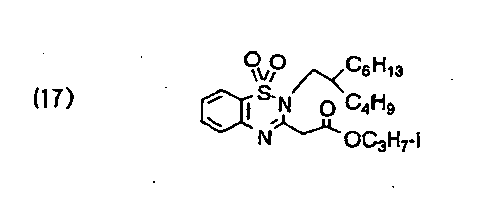

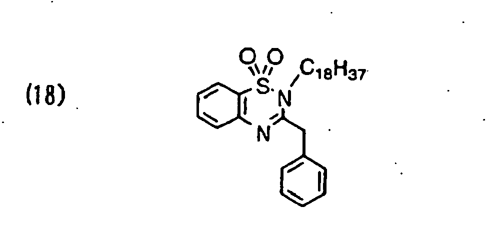

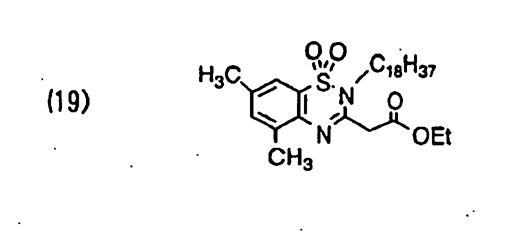

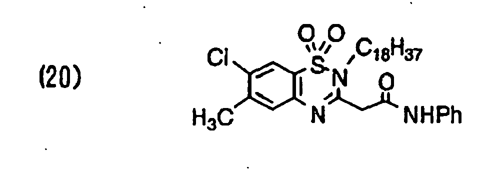

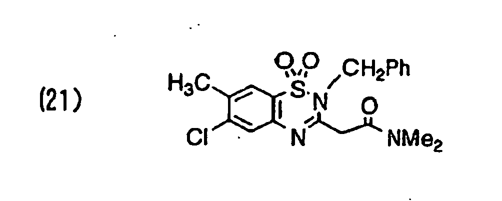

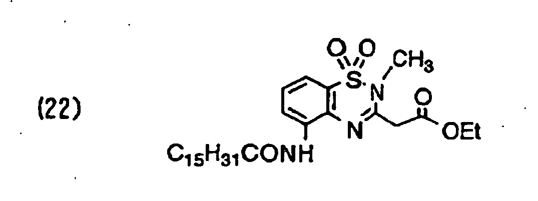

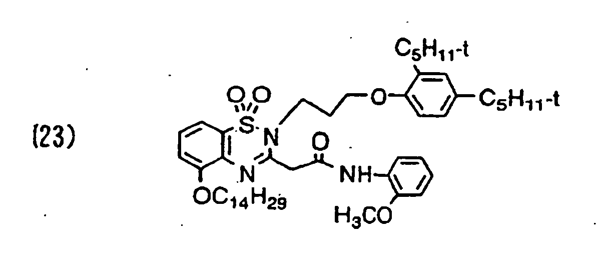

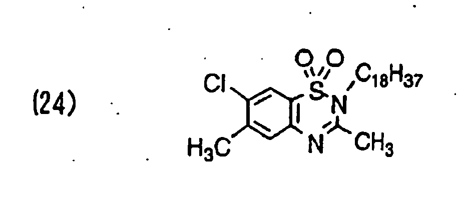

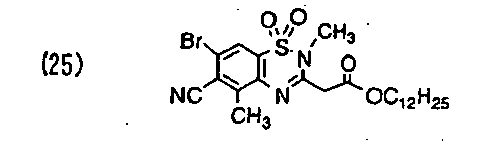

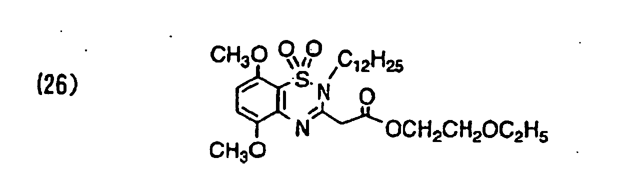

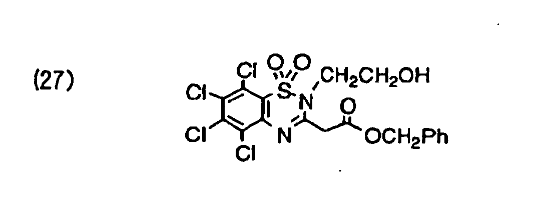

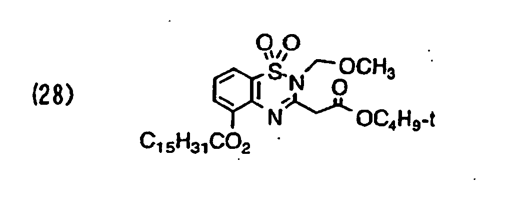

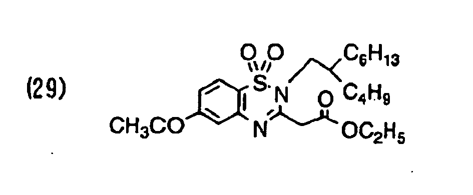

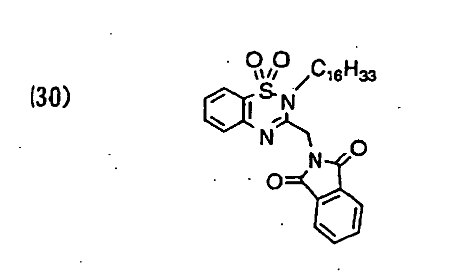

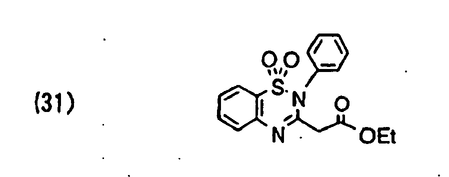

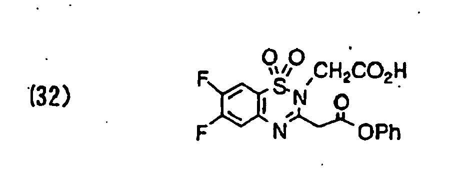

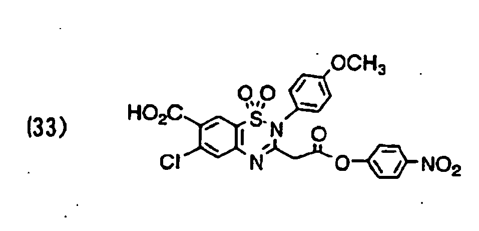

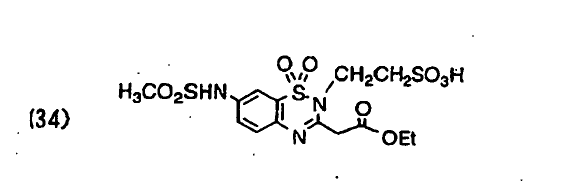

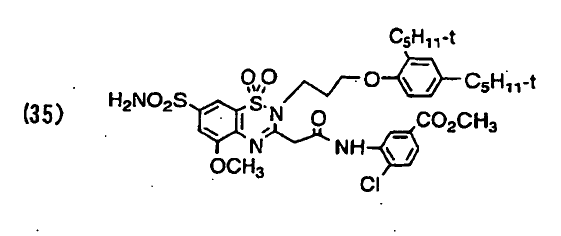

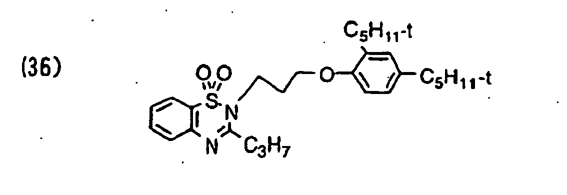

Specific examples of the compounds represented by formula (II) are shown below, but the present invention should not be construed to be limited to them.

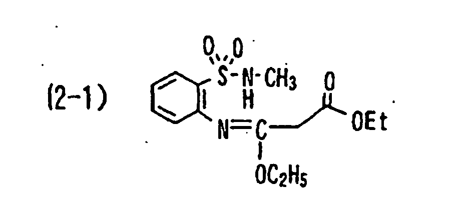

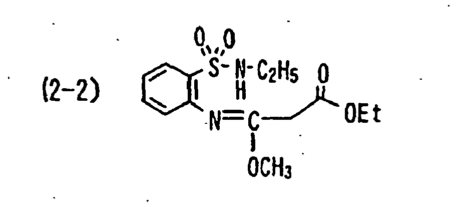

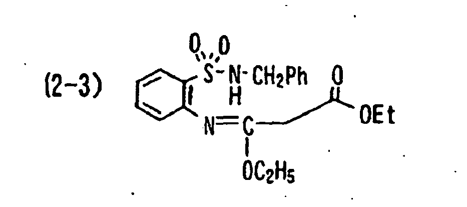

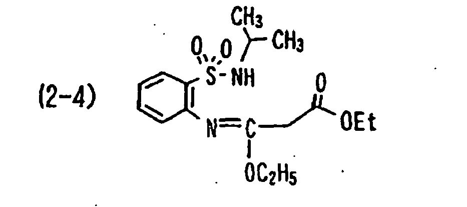

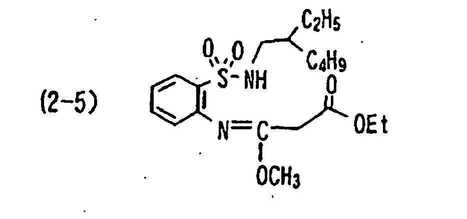

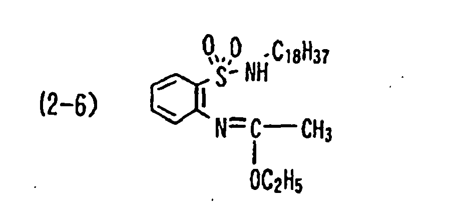

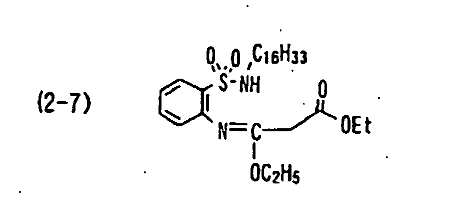

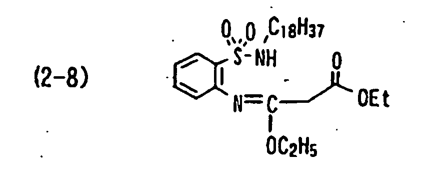

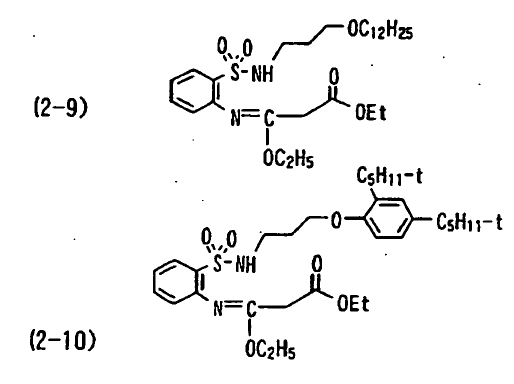

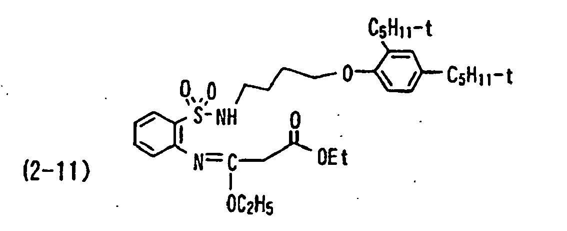

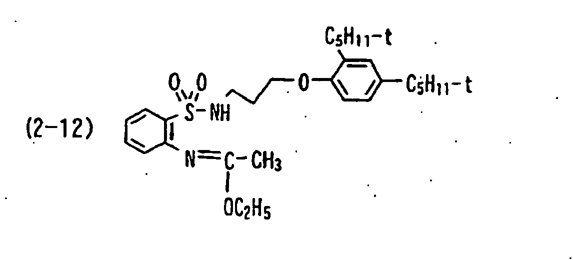

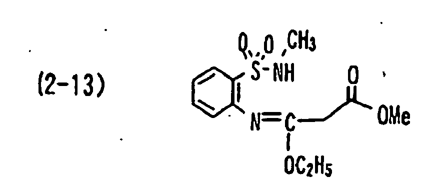

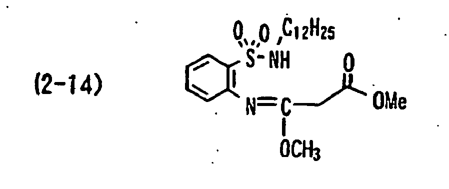

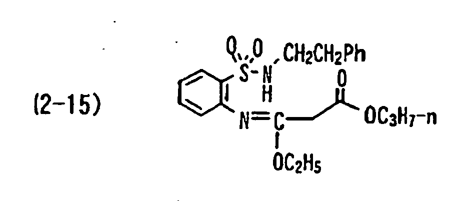

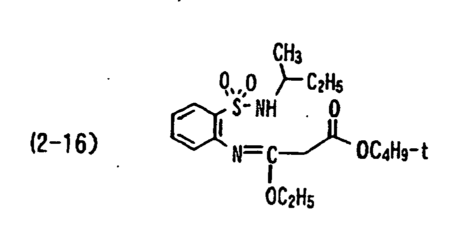

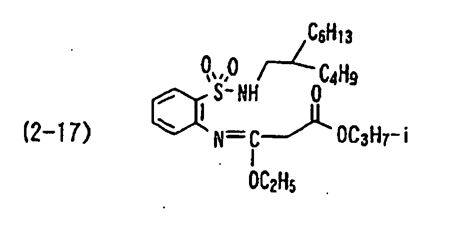

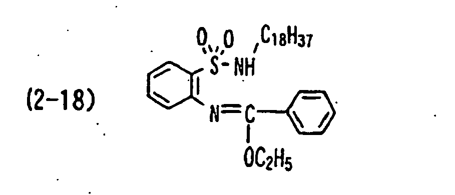

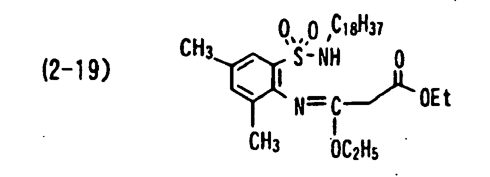

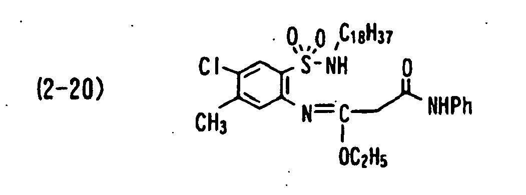

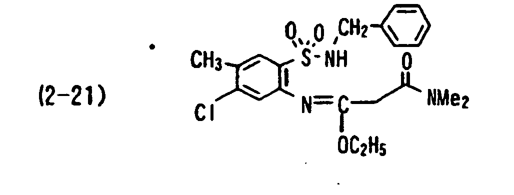

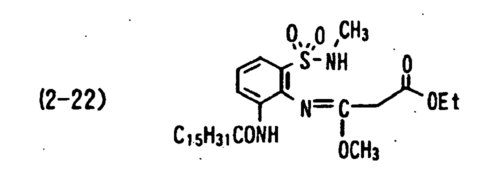

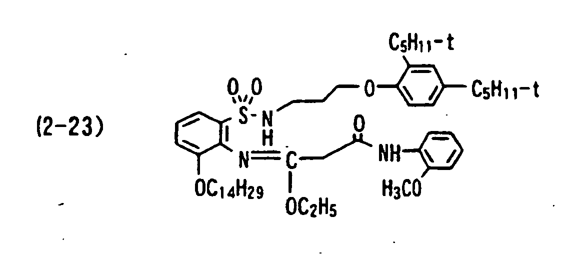

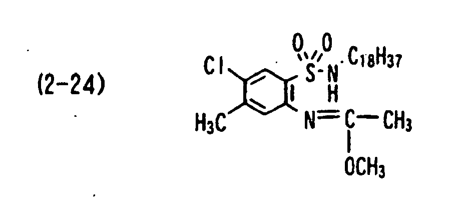

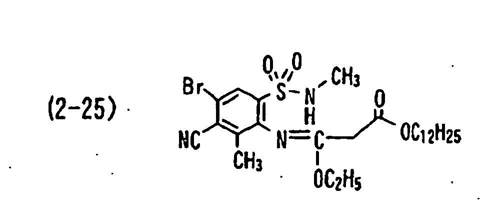

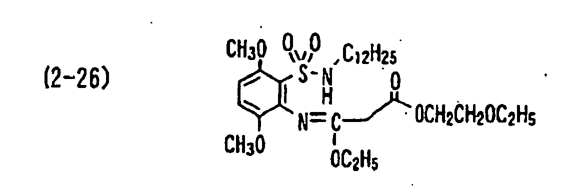

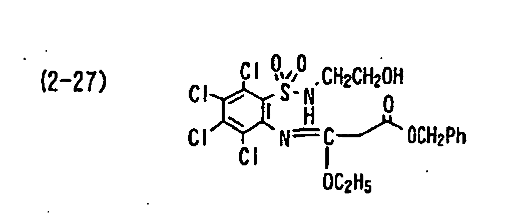

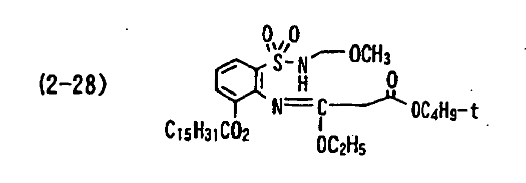

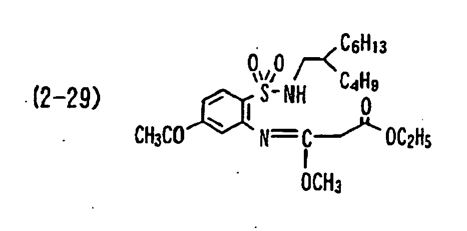

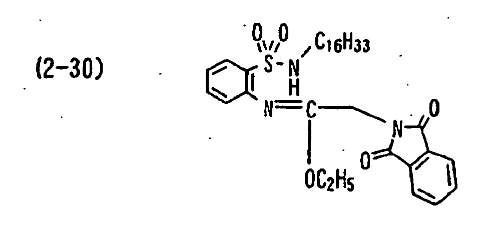

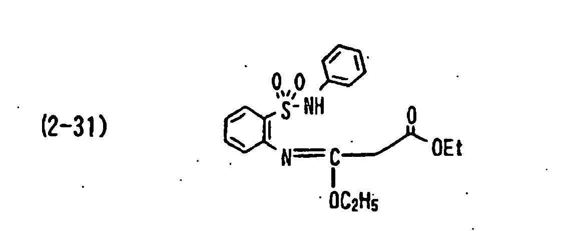

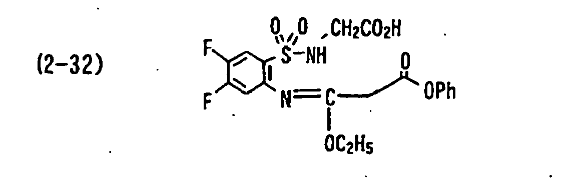

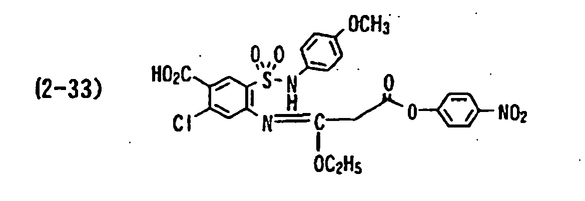

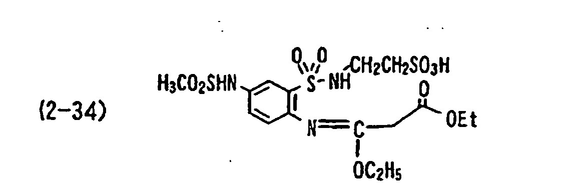

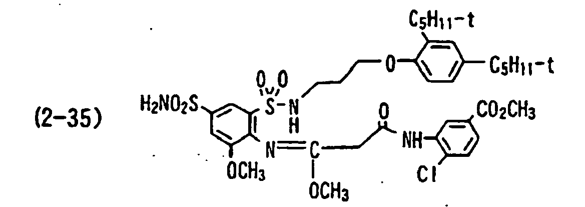

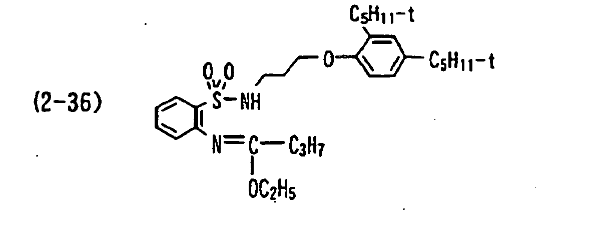

Specific example of the compound represented by formula (III) can be specified from the specific examples of the compound represented by formula (II) shown above. Namely, those compounds in which the -N=C(OR₄)CH₂R₃ moiety in formula (II) is replaced with a -NH₂ moiety. The present invention is not limited to these compounds.

Specific examples of the compounds represented by formula (IV) or formula (V) are shown below, but the present invention should not be construed to be limited to them. 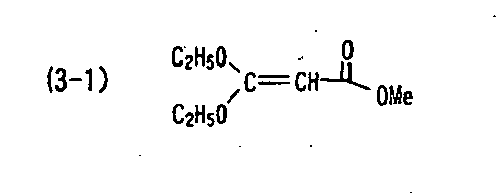 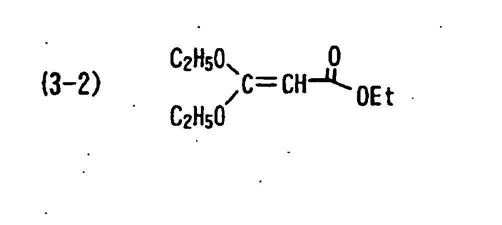 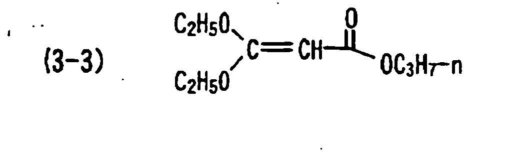 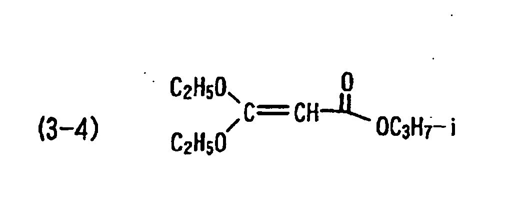 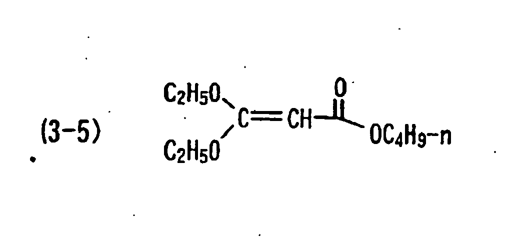 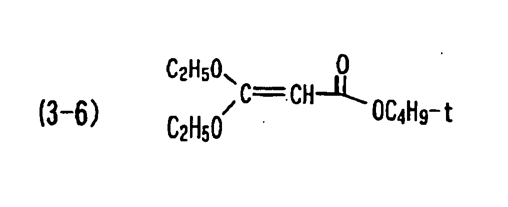 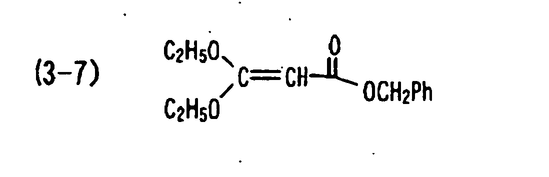 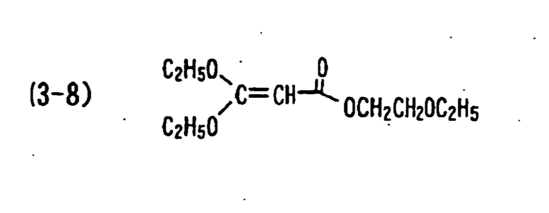 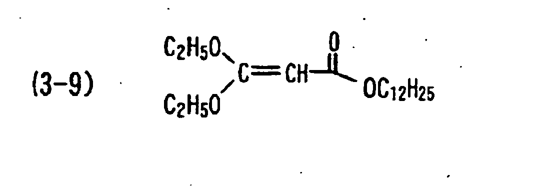 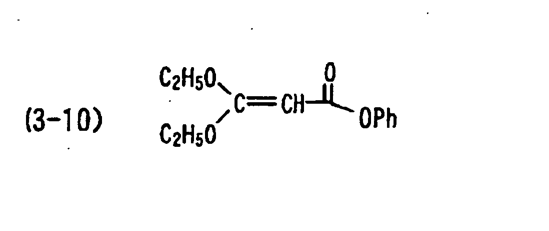 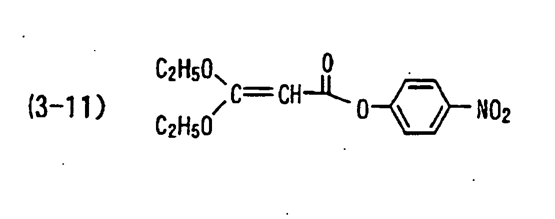 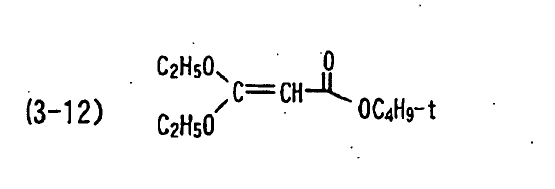 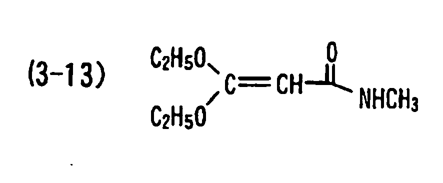 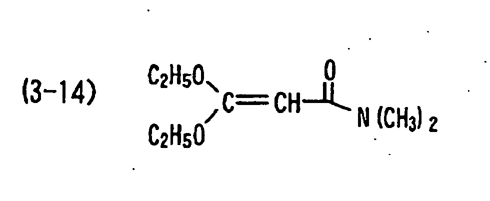 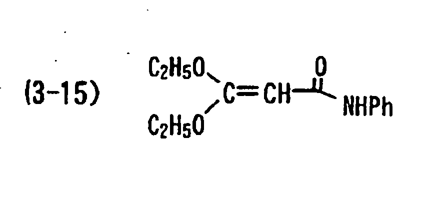 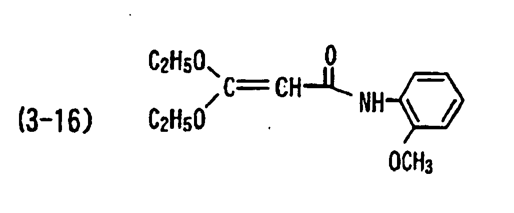 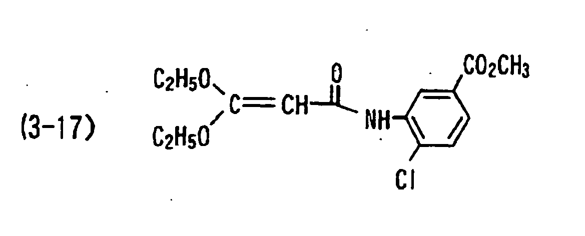 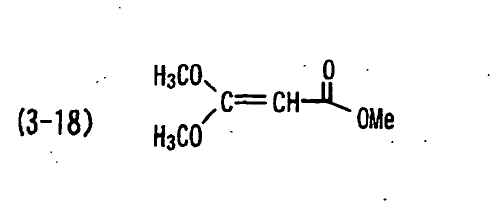 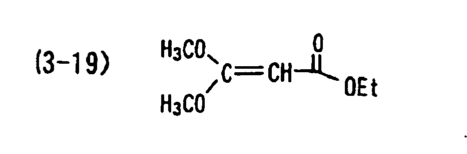 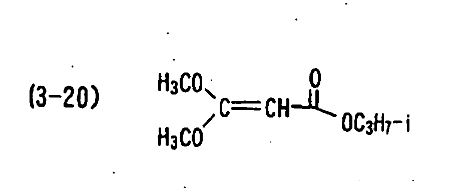 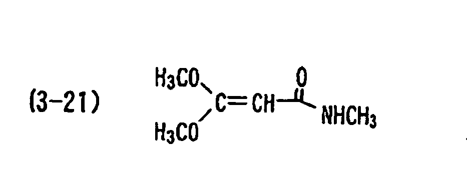 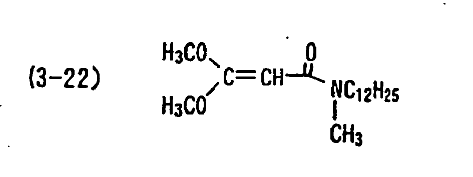 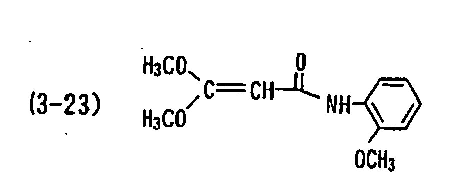 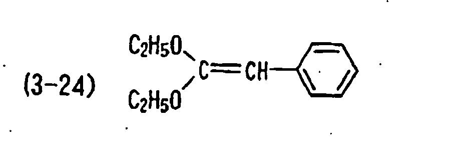 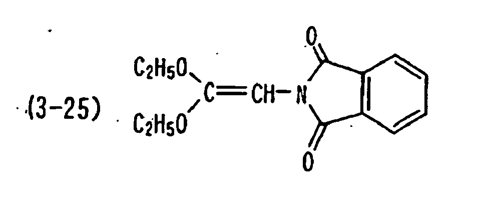 (3-26) CH₃-C-(OCH₃)₃

(3-27) CH₃-C-(OC₂H₅)₃

(3-28) C₂H₅-C-(OC₂H₅)₃

(3-29) j-C₃H₇-C-(OC₂H₅)₃

(3-30) C₁₂H₂₅-C-(OC₂N₅)₃

In the following explanation, when the exemplified compounds shown above are referred to, the number X in the parenthesis, i.e., (X), is labeled to each of the exemplified compounds, and they are expressed as "exemplified compound (X)".

In the present invention, the compound represented by formula (I) may be synthesized by a successive reaction in one pot without isolating the produced intermediate represented by formula (II). Alternatively, the compound represented by formula (I) may be synthesized by a method, in which once the intermediate represented by formula (II) is separated, or once a reaction mixture of said intermediate is prepared by an operation such as extraction and concentration, then the intermediate or the reaction mixture is subjected to a reaction under another condition to obtain an objective compound.

The compound represented by formula (III) and the compound represented by formula (IV) or (V) that are used in the present invention can be synthesized by various known methods.

The compound represented by formula (III) can be readily synthesized, most generally, by a method in which an ortho-nitrobenzenesulfonyl chloride compound is reacted with an amine, and then the nitro group is reduced.

The compound represented by formula (IV) or (V) can be readily synthesized, most generally, by a method in which a nitrile is subjected to a reaction in a solvent, such as an ether, in the presence of an alcohol and an acid.

In the production method of present invention, a course of production from the first stage of synthesizing the compound represented by formula (III) to the final stage of synthesizing the compound represented by formula (I) itself can be carried out with no isolation of an intermediate that is produced during the course, and/or with simply using a reaction mixture that is obtained by an operation such as extraction or concentration of a reaction solution.

Specifically, after completion of a reaction between an ortho-nitrobenzene sulfonyl chloride compound and an amine, both of which are starting materials for synthesis of the compound represented by formula (III), the nitro group is reduced without isolation of the reaction product obtained in this step. Successively, without isolation of the thus-produced aniline compound represented by formula (III), the aniline compound is reacted with a compound represented by formula (IV) or (V). Further successively, without isolation of the produced intermediate represented by formula (II), the intermediate may be subjected to a certain reaction in one pot (i.e., the same reactor) to obtain the compound represented by formula (I). Alternatively, once the intermediate represented by formula (II) is isolated, or once a reaction mixture of the intermediate is prepared by an operation such as extraction or concentration, and then said intermediate or said reaction mixture may be subjected to a reaction under another condition to obtain the compound represented by formula (I).

For enhancement of productivity and reduction in production cost, it is preferable that all the reaction steps are carried out in one pot.

The compound represented by formula (III) and the compound represented by formula (IV) or (V) are used generally in a molar ratio of 10:1 to 1:100, preferably in a molar ratio of 10:1 to 1:10, further preferably in a molar ratio of 5:1 to 1:5, and especially preferably in a molar ratio of 1:1 to 1:3.

In the present invention, a reaction may be conducted with no solvent, but preferably a reaction may be performed in the form of solution or dispersion with the aid of a proper solvent. Examples of the solvent that can be used in the present invention include water, alcohol-series solvents (such as methanol, isopropanol), chlorine-series solvents (such as dichloromethane, chloroform), aromatic-series solvents (such as benzene, toluene, xylene, chlorobenzene), amide-series solvents (such as N,N-dimethylformamide, N,N-dimethyl acetoamide), ureide-series solvents (such as 1,3-dimethyl-2-imidazolidinone), nitrile-series solvents (such as acetonitrile), ether-series solvents (such as diethylether, tetrahydrofran), sulfon-series solvents (such as sulfolane), sulfoxide-series solvents (such as dimethylsulfoxide), phosphoric amide-series solvents (such as hexamethyl phosphoric triamide), and hydrocarbon-series solvents (such as hexane, cyclohexane).

These solvents may be used singly or as a mixture of two or more solvents. The solvent is used in a proportion of preferably 0.1 to 1000 mass parts, more preferably 0.5 to 100 mass parts, and still further preferably 1 to 50 mass parts, per mass part of the compound represented by formula (III), or the intermediate represented by formula (II).

When the compound represented by formula (III) is reacted with the compound represented by formula (IV) or (V), it is preferable to use an aromatic-series solvent, more preferably toluene or xylene. Also in the step in which the compound represented by formula (I) is synthesized from the intermediate represented by formula (II), an aromatic-series solvent is preferably used. Toluene and xylene are more preferable.

Examples of the acid that can be used in the reaction between the compound represented by formula (III) and the compound represented by formula (IV) or (V) include inorganic acids, such as hydrogen chloride, hydrogen bromide, hydrogen iodide, sulfuric acid, and carbonic acid; organic carboxylic acids, such as acetic acid, monochloroacetic acid, dichloroacetic acid, trichloroacetic acid, propionic acid, butyric acid, oxalic acid, maleic acid, benzoic acid, salicylic acid, and phthalic acid; organic sulfonic acids, such as methane sulfonic acid, ethane sulfonic acid, benzene sulfonic acid, and p-toluene sulfonic acid; Lewis acids, such as boron trifluoride, aluminum chloride, and zinc chloride, and the like. Among them, sulfuric acid, acetic acid, methane sulfonic acid, ethane sulfonic acid, and p-toluene sulfonic acid are preferred, and a proper acid should be selected from among them according to the substrate.

In the reaction between the compound represented by formula (III) and the compound represented by formula (IV) or (V), the compound represented by formula (III) and the acid are used generally in a molar ratio of 1:0.001 to 1:100, preferably in a molar ratio of 1:0.001 to 1:50, further preferably in a molar ratio of 1:0.001 to 1:10, and especially preferably in a molar ratio of 1:0.001 to 1:3.

Examples of the base that can be used in the reaction, upon which the compound represented by formula (I) is synthesized from the intermediate represented by formula (II), include organic bases (such as triethylamine, tributylamine, 1,8-diazabicyclo[5,4,0]-7-undecene (DBU), 1,5-diazabicyclo[4,3,0]-5-nonene (DBN), 1,4-diazabicyclo[2,2,2]octane), metal hydrides (such as sodium hydride, potassium hydride), metal alkoxides (such as sodium methoxide, sodium ethoxide, potassium t-butoxide, sodium t-butoxide), metal hydroxides (such as potassium hydroxide, sodium hydroxide, calcium hydroxide), carbonic acid bases (such as potassium carbonate, sodium carbonate, sodium hydrogencarbonate), and carboxylic acid bases (such as sodium acetate). Preferred among them are 1,8-diazabicyclo[5,4,0]-7-undecene (DBU), sodium hydride, potassium hydride, sodium methoxide, sodium ethoxide, potassium t-butoxide, and sodium t-butoxide. A proper base should be selected from among them according to the substrate.

In the ring closure reaction of the compound represented by formula (II) in the presence of a base, the compound of formula (II) and the base are used generally in a molar ratio of 1:0.001 to 1:100, preferably in a molar ratio of 1:0.01 to 1:50, further preferably in a molar ratio of 1:0.01 to 1:10, and especially preferably in a molar ratio of 1:0.01 to 1:3.

In the reaction between the compound represented by formula (III) and the compound represented by formula (IV) or (V), the reaction temperature is not particularly limited, and the reaction can be conducted generally in the range of 0 °C to 300 °C, preferably in the range of 10 °C to 250 °C, and further preferably in the range of 20 °C to 200 °C. In the step in which the compound represented by formula (I) is synthesized from the intermediate represented by formula (II), the reaction temperature is not particularly limited, and the reaction can be conducted generally in the range of 0 °C to 300 °C, preferably in the range of 10 °C to 250 °C, and further preferably in the range of 20 °C to 200 °C. A proper reaction temperature should be selected from among the ranges according to the substrate.

In the reaction between the compound represented by formula (III) and the compound represented by formula (IV) or (V), the reaction can be conducted with the reaction time of generally in the range of 1 minute to 50 hours, and preferably in the range of 1 minute to 20 hours, and further preferably in the range of 1 minute to 5 hours. In the reaction upon which the compound represented by formula (I) is synthesized from the intermediate represented by formula (II), the reaction can be conducted with the reaction time of generally in the range of 10 minutes to 50 hours, preferably in the range of 10 minutes to 20 hours, and further preferably in the range of 10 minutes to 10 hours. A proper reaction time should be selected from among the ranges according to the substrate.

According to the present invention, it is possible to provide a method of producing 1,2,4-benzothiadiazine-1,1-dioxide compounds from readily obtainable raw materials, in short steps, under mild reaction conditions, with a good yield.

Hereinafter, the present invention will be described in more detail by way of examples, but the present invention should not be limited thereto.

### EXAMPLES

### (Example 1) Synthesis of Exemplified Compound (8)

Exemplified Compound (8) was synthesized in accordance with the following scheme.

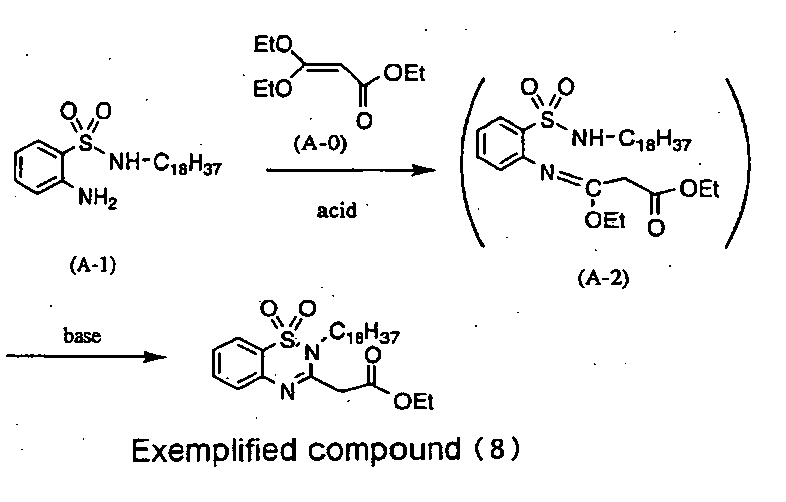

20 ml of toluene was added to a mixture of 10.0 g (23.6 mmol) of compound (A-1), 5.32 g (28.3 mmol) of ethyl 3,3-diethoxyacrylate (A-0) and 0.045 g (0.24 mmol) of p-toluene sulfonic acid monohydrate, and heated under reflux for 30 minutes. After adding 0.226 g (2.4 mmol) of sodium t-butoxide, the reaction solution was heated under reflux for 5 hours. After the reaction solution had been cooled to room temperature, the solution was neutralized with dilute hydrochloric acid. Then, 30 ml of ethyl acetate was added. The organic solvent layer was washed with water, and dried over sodium sulfate. Thereafter, the solvent was removed by vacuum distillation. The resulting product was crystallized from methanol, to obtain 11.2 g of an objective compound identified as exemplified compound (8). Yield 91.1%. m.p.: 80 to 81 °C. ¹H-NMR (300MHz, CDCl₃) 11.72 (s, 1H), 7.78 (d, 1H), 7.58 (t, 1H), 7.19 (t, 1H), 7.10 (d, 1H), 4.61 (s, 1H), 4.20 (q, 2H), 2.27 (s, 3H), 1.32 (t, 3H), (enamine form).

### (Example 2) Synthesis of Exemplified Compound (8)

20 ml of xylene was added to a mixture of 8.49 g (20 mmol) of compound (A-1), 4.54 g (24 mmol) of ethyl 3,3-diethoxyacrylate (A-0), and 0.60 g (6.52 mmol) of acetic acid, and heated under reflux for 30 minutes. Further, 3.04 g (20 mmol) of DBU was added and heated under reflux for 7 hours. After the reaction solution had been cooled to room temperature, the solution was neutralized with dilute hydrochloric acid, and then, 30 ml of ethyl acetate was added. After washing with water, the organic solvent layer was dried over sodium sulfate. The solvent was removed by vacuum distillation. The resulting product was crystallized from methanol, to obtain 8.78 g of an objective compound identified as exemplified compound (8). Yield 84.0%

### (Example 3) Synthesis of Exemplified Compound (8)

Exemplified Compound (8) was synthesized in accordance with the following scheme.

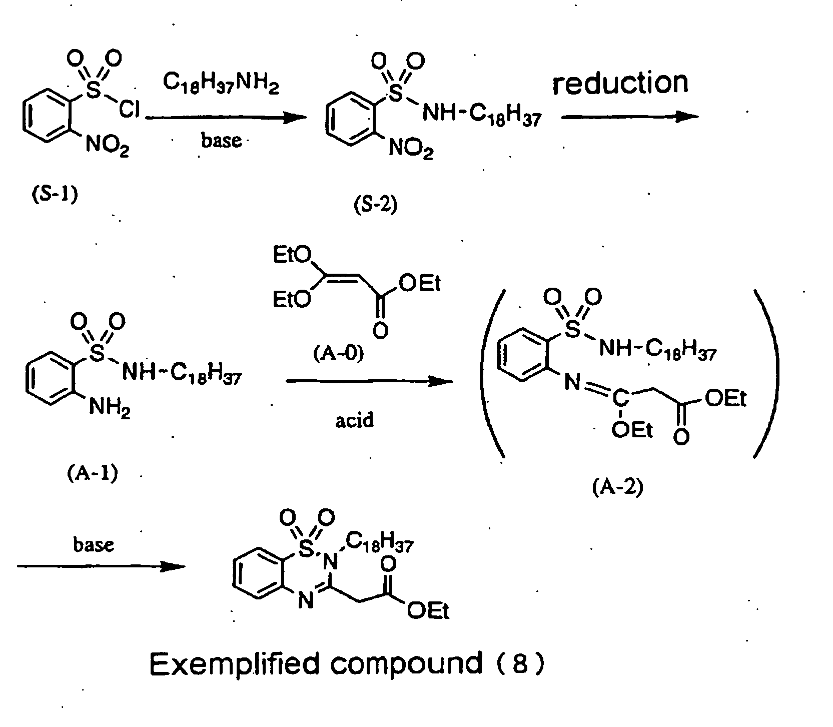

150 ml of xylene was added to a mixture of 30 g (0.111 mol) of octadecylamine and 15.8 ml (0.113 mol) of triethylamine, and heated to 30 °C. To the solution, 24.7 g (0.111 mol) of orthonitrobenzenesulfonyl chloride (S-1) was added by way of divided addition. Owing to the exothermic reaction, the temperature of the reaction solution elevated up to 50 °C. Further, the reaction solution was stirred at 60 °C for 2 hours, and then washed twice with warm water, to obtain a xylene solution containing Compound (S-2). The resulting solution was placed in 500 ml autoclave, and 30 ml of ethanol and 5 g of Ra-Ni (Raney Nickel) were added. After filling with 50 kg/cm² of hydrogen gas, the reaction mixture was stirred at 80 °C for 2 hours. After confirmation of consumption in a theoretical amount of the hydrogen pressure, the catalyst was removed by vacuum filtration. Ethanol in the filtrate was removed by vacuum distillation. To the resulting residue, 25.1 g (0.133 mol) of ethyl 3,3-diethoxyacrylate (A-0) and 0.212 g (1.11 mmol) of p-toluene sulfonic acid monohydrate were added, and heated under reflux for 30 minutes. Further, 1.07 g (1.11 mmol) of sodium t-butoxide was added, and then heated under reflux for 5 hours. After the reaction solution had been cooled to room temperature, the solution was neutralized with dilute hydrochloric acid. Then, 150 ml of ethyl acetate was added. The organic solvent layer was washed with water, and dried over sodium sulfate. Thereafter, the solvent was removed by vacuum distillation. The resulting product was crystallized from methanol, to obtain 51.0 g of an objective compound identified as exemplified compound (8). Yield 87.9%

### (Example 4) Synthesis of Exemplified Compound (10)

Exemplified Compound (10) was synthesized in accordance with the following scheme.

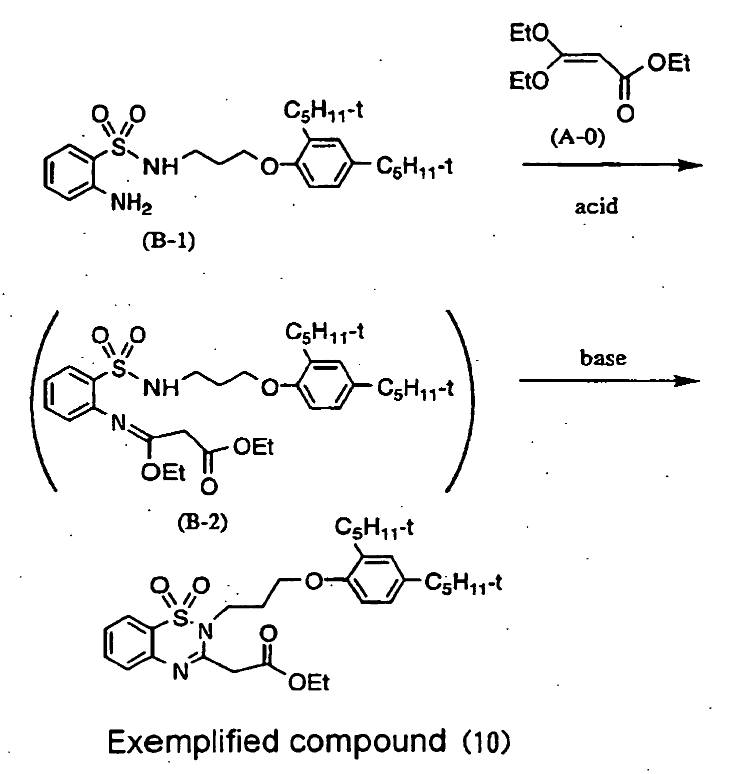

20 ml of toluene was added to a mixture of 10.0 g (22.4 mmol) of compound (B-1), 5.06 g (26.9 mmol) of ethyl 3,3-diethoxyacrylate (A-0) and 0.043 g (0.224 mmol) of p-toluene sulfonic acid monohydrate, and heated under reflux for 30 minutes. Further, 0.215 g (2.24 mmol) of sodium t-butoxide was added, and then heated under reflux for 5 hours. After the reaction solution had been cooled to room temperature, the solution was neutralized with dilute hydrochloric acid. Then, 20 ml of ethyl acetate was added. The organic solvent layer was washed with water and dried over sodium sulfate. Thereafter, the solvent was removed by vacuum distillation. The resulting product was crystallized from methanol, to obtain 10.1 g of an objective compound identified as exemplified compound (10).
Yield 83.1%. m.p.: 76 to 77 °C. ¹H-NMR (300MHz, CDCl₃) 11.82 (s, 1H), 7.77 (d, 1H), 7.56 (t, 1H), 7.22 to 7.05 (m, 4H), 6.73 (d, 1H), 4.80 (s, 1H), 4.20 (q, 2H), 4.10 to 3.98 (m, 4H), 2.38 to 2.26 (m, 2H), 1.87 (q, 2H), 1.60 (q, 2H), 1.39 (s, 6H), 1.30 (t, 3H), 1.26 (s, 6H), 0.65 (q, 6H), (enamine form).

### (Example 5) Synthesis of Exemplified Compound (6)

Exemplified Compound (6) was synthesized in accordance with the following scheme.

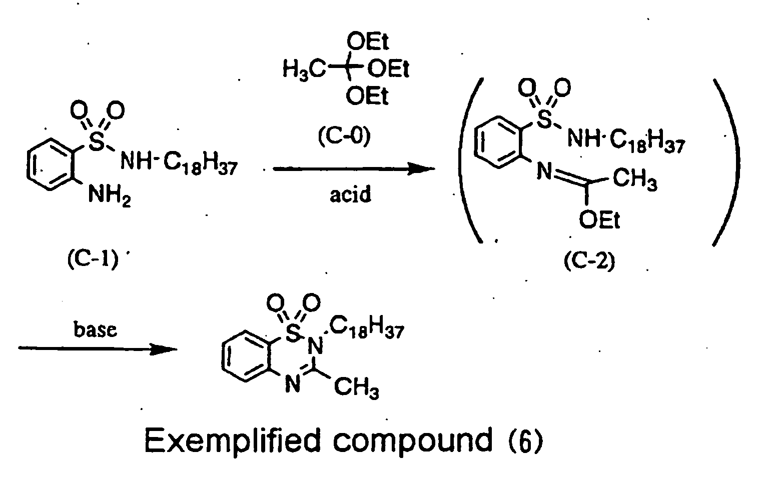

10 ml of toluene was added to a mixture of 4.25 g (10.0 mmol) of compound (C-1), 1.44 g (12.0 mmol) of ethyl orthoacetate (C-0) and 0.019 g (0.1 mmol) of p-toluene sulfonic acid monohydrate, and then heated under reflux for 1 hour. Further, 0.096 g (1.0 mmol) of sodium t-butoxide was added, and then heated under reflux for 3 hours. After the reaction solution had been cooled to room temperature, the solution was neutralized with dilute hydrochloric acid. Then, 20 ml of ethyl acetate was added. The organic solvent layer was washed with water, and dried over sodium sulfate. Thereafter, the solvent was removed by vacuum distillation. The resulting product was crystallized from methanol, to obtain 3.6 g of an objective compound identified as exemplified compound (6). Yield 80.2%. m.p.: 47 to 48 °C. ¹H-NMR (300MHz, CDCl₃), 7.85 (d, 1H), 7.60 to 7.70 (m, 1H), 7.38 to 7.52 (m, 2H), 3.81 to 3.95 (m, 2H), 2.51 (s, 3H), 1.69 to 1.81 (m, 2H), 1.13 to 1.50 (m, 30H), 0.89 (t, 3H).

### (Example 6) Synthesis of Exemplified Compounds (7), (9), (12), and (14)

Exemplified compounds (7), (9), and (14) were also obtained with high yield according to the method described in Example 1 by changing the raw materials to corresponding ones. Similarly, exemplified compound (12) was also obtained with high yield according to the method described in Example 5 by changing the raw materials to corresponding ones.

## Claims

1. A method of producing a 1,2,4-benzothiadiazine-1,1-dioxide compound represented by formula (I), comprising subjecting an iminoether compound represented by formula (II) to a ring closure reaction in the presence of a base: 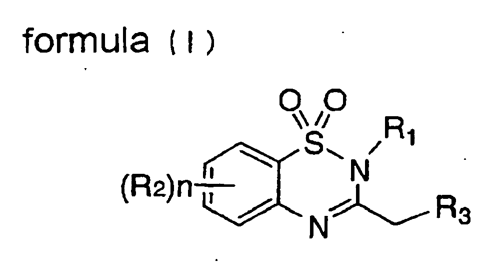 wherein, in formula (I), R₁ represents a substituted or unsubstituted alkyl group, or a substituted or unsubstituted aryl group; R₂ represents a substituent; n represents an integer of 0 to 4; when n is 2 or more, R₂s may be the same or different, and R₂s may bond with each other to form a ring; and R₃ represents a hydrogen atom or a substituent; 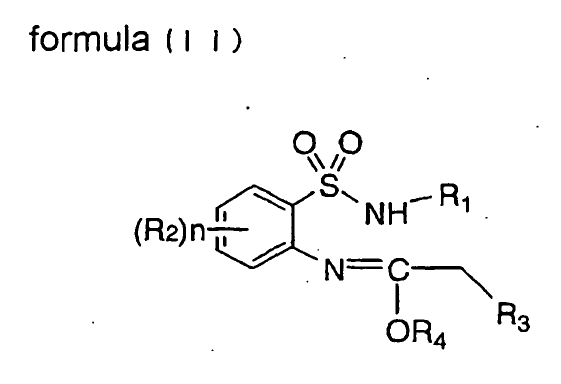 wherein, in formula (II), R₁ represents a substituted or unsubstituted alkyl group, or a substituted or unsubstituted aryl group; R₂ represents a substituent; n represents an integer of 0 to 4; when n is 2 or more, R₂s may be the same or different, and R₂s may bond with each other to form a ring; R₃ represents a hydrogen atom or a substituent; and R₄ represents a substituted or unsubstituted alkyl group.

2. The method as claimed in claim 1, wherein the base is an organic base, a metal hydride, a metal alkoxide, a metal hydroxide, a carbonic acid base, or a carboxylic acid base.

3. The method as claimed in claim 1, wherein, in the ring closure reaction of the compound represented by formula (II), the compound of formula (II) and the base are used in a molar ratio of 1:0.001 to 1:100.

4. A method of producing a 1,2,4-benzothiadiazine-1,1-dioxide compound represented by formula (I) of claim 1, comprising reacting a 2-aminobenzenesulfonamide compound represented by formula (III) with a 1,1-dialkoxyalkene compound represented by formula (IV) or with an orthoester compound represented by formula (V), in the presence of an acid, to obtain an iminoether compound represented by formula (II) of claim 1, and then subjecting the iminoether compound to a ring closure reaction, in the presence of a base: 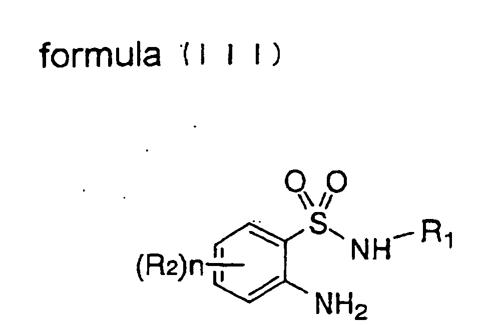 wherein, in formulae (I) to (III), R₁ to R₃ are the same as in claim 1; 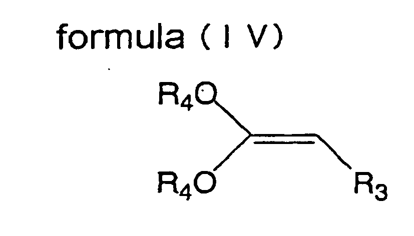 wherein, in formula (IV), R₃ represents a hydrogen atom or a substituent; and R₄ represents a substituted or unsubstituted alkyl group; and 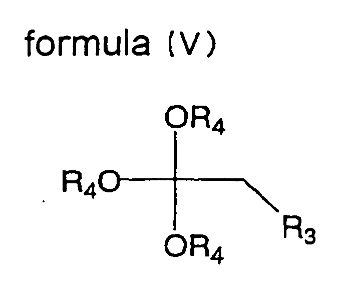 wherein, in formula (V), R₃ represents a hydrogen atom or a substituent; and R₄ represents a substituted or unsubstituted alkyl group.

5. The method as claimed in claim 4, wherein R₁ is an alkyl group having 1 to 60 carbon atoms in total, or an aryl group having 6 to 60 carbon atoms in total.

6. The method as claimed in claim 4, wherein R₃ is a hydrogen atom, an alkyl group, an aryl group, an alkoxycarbonyl group, an aryloxycarbonyl group, or a carbamoyl group.

7. The method as claimed in claim 4, wherein the acid is an inorganic acid, an organic carboxylic acid, an organic sulfonic acid, or a Lewis acid.

8. The method as claimed in claim 4, wherein the base is an organic base, a metal hydride, a metal alkoxide, a metal hydroxide, a carbonic acid base, or a carboxylic acid base.

9. The method as claimed in claim 4, wherein, in the reaction between the compound represented by formula (III) and the compound represented by formula (IV) or (V), the compound represented by formula (III) and the acid are used in a molar ratio of 1:0.001 to 1:100.

10. The method as claimed in claim 4, wherein, in the ring closure reaction of the compound represented by formula (II), the compound of formula (II) and the base are used in a molar ratio of 1:0.001 to 1:100.

11. The method as claimed in any of claims 1 to 3, wherein R₁ in formulae (I) and (II) represents a substituted or unsubstituted alkyl group.

12. The method as claimed in any of claims 4 to 10, wherein R₁ in formulae (I), (II) and (III) represents a substituted or unsubstituted alkyl group.

13. The method as claimed in any of the preceding claims, wherein the reaction solvent is toluene or xylene.

14. The method as claimed in claim 13, wherein the reaction solvent is toluene.

## Patentansprüche

1. Verfahren zur Herstellung einer durch die Formel (I) dargestellten 1,2,4-Benzothiadiazin-1,1-dioxidverbindung, das eine Ringschlußreaktion einer durch die Formel (II) dargestellten Iminoetherverbindung in Gegenwart einer Base umfaßt: 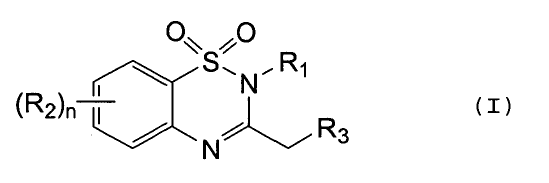 worin in der Formel (I) R₁ eine substituierte oder unsubstituierte Alkylgruppe oder eine substituierte oder unsubstituierte Arylgruppe darstellt; R₂ stellt einen Substituenten dar; n stellt eine ganze Zahl von 0 bis 4 dar; wenn n 2 oder mehr ist, können die R₂s gleich oder unterschiedlich sein, und die R₂s können miteinander verbunden sein, um einen Ring zu bilden; und R₃ stellt ein Wasserstoffatom oder einen Substituenten dar; 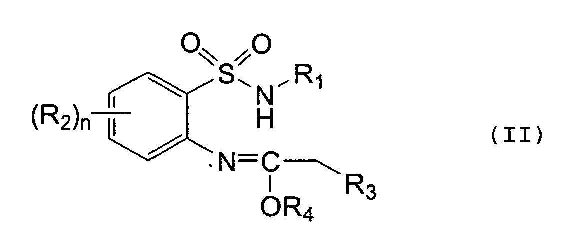 worin in Formel (II) R₁ eine substituierte oder unsubstituierte Alkylgruppe oder eine substituierte oder unsubstituierte Arylgruppe darstellt; R₂ stellt einen Substituenten dar; n stellt eine ganze Zahl von 0 bis 4 dar; wenn n 2 oder mehr ist, können die R₂s gleich oder unterschiedlich sein und die R₂s können miteinander verbunden sein, um einen Ring zu bilden; R₃ stellt ein Wasserstoffatom oder einen Substituenten dar; und R₄ stellt eine substituierte oder unsubstituierte Alkylgruppe dar.

2. Verfahren gemäß Anspruch 1, worin die Base eine organische Base, ein Metallhydrid, ein Metallalkoxid, ein Metallhydroxid, eine Kohlensäurebase oder eine Carbonsäurebase ist.

3. Verfahren gemäß Anspruch 1, worin bei der Ringschlußreaktion der durch die Formel (II) dargestellten Verbindung die Verbindung der Formel (II) und die Base in einem molaren Verhältnis von 1:0,001 bis 1:100 verwendet werden.

4. Verfahren zur Herstellung einer durch die Formel (I) dargestellten 1,2,4-Benzothiadiazin-1,1-dioxidverbindung gemäß Anspruch 1, das die Reaktion einer durch die Formel (III) dargestellten 2-Aminobenzolsulfonamidverbindung mit einer durch die Formel (IV) dargestellten 1,1-Dialkoxyalkenverbindung oder mit einer durch die Formel (V) dargestellten Orthoesterverbindung in der Gegenwart einer Säure, um eine durch die Formel (II) des Anspruchs 1 dargestellte Iminoetherverbindung zu erhalten, und dann Durchführen der Ringschlußreaktion der Iminoetherverbindung in der Gegenwart einer Base umfaßt. 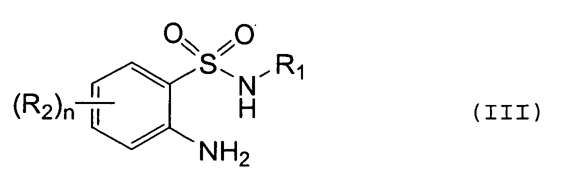 worin in Formeln (I) bis (III) R₁ bis R₃ die gleichen wie in Anspruch 1 sind; 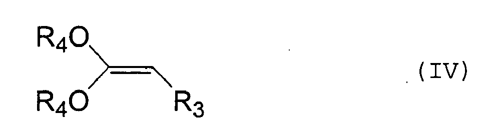 worin in Formel (IV) R₃ ein Wasserstoffatom oder einen Substituenten darstellt; und R₄ stellt eine substituierte oder unsubstituierte Alkylgruppe dar; und 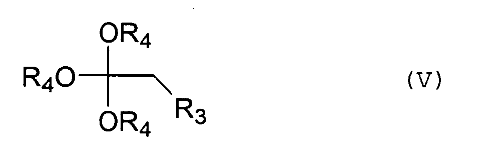 worin in Formel (V) R₃ ein Wasserstoffatom oder einen Substituenten darstellt und R₄ stellt eine substituierte oder unsubstituierte Alkylgruppe dar.

5. Verfahren gemäß Anspruch 4, worin R₁ eine Alkylgruppe mit insgesamt 1 bis 60 Kohlenstoffatomen oder eine Arylgruppe mit insgesamt 6 bis 60 Kohlenstoffatomen ist.

6. Verfahren gemäß Anspruch 4, worin R₃ ein Wasserstoffatom, eine Alkylgruppe, eine Arylgruppe, eine Alkoxycarbonylgruppe, eine Aryloxycarbonylgruppe oder eine Carbamoylgruppe ist.

7. Verfahren gemäß Anspruch 4, worin die Säure eine anorganische Säure, eine organische Carbonsäure, eine organische Sulfonsäure oder eine Lewis-Säure ist.

8. Verfahren gemäß Anspruch 4, worin die Base eine organische Base, ein Metallhydrid, ein Metallalkoxid, ein Metallhydroxid, eine Kohlensäurebase oder eine Carbonsäurebase ist.

9. Verfahren gemäß Anspruch 4, worin bei der Reaktion zwischen der durch die Formel (III) dargestellten Verbindung und der durch die Formel (IV) oder (V) dargestellten Verbindung die durch die Formel (III) dargestellte Verbindung und die Säure in einem molaren Verhältnis von 1:0,001 bis 1:100 verwendet werden.

10. Verfahren gemäß Anspruch 4, worin bei der Ringschlußreaktion der durch die Formel (II) dargestellten Verbindung die Verbindung der Formel (II) und die Base in einem molaren Verhältnis von 1:0,001 bis 1:100 verwendet werden.

11. Verfahren gemäß irgendeinem der Ansprüche 1 bis 3, worin R₁ in Formeln (I) und (II) eine substituierte oder unsubstituierte Alkylgruppe darstellt.

12. Verfahren gemäß irgendeinem der Ansprüche 4 bis 10, worin R₁ in Formeln (I), (II) und (III) eine substituierte oder unsubstituierte Alkylgruppe darstellt.

13. Verfahren gemäß irgendeinem der vorhergehenden Ansprüche, worin das Reaktionslösungsmittel Toluol oder Xylo1 ist.

14. Verfahren gemäß Anspruch 13, worin das Reaktionslösungsmittel Toluol ist.

## Revendications

1. Procédé de production d'un composé 1,2,4-benzothiadiazine-1,1-dioxyde représenté par la formule (I), comprenant de soumettre un composé iminoéther représenté par la formule (II) à une réaction de cyclisation en présence d'une base : 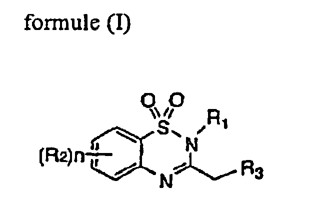 dans laquelle, dans la formule (I), R₁ représente un groupe alkyle substitué ou non substitué, ou un groupe aryle substitué ou non substitué ; R₂ représente un substituant ; n représente un entier de 0 à 4 ; quand n est 2 ou plus, R₂s peuvent être identiques ou différents, et R₂s peuvent se liés les uns aux autres pour former un cycle ; et R₃ représente un atome d'hydrogène ou un substituant ; 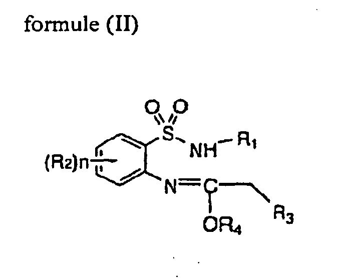 dans laquelle, dans la formule (II), R₁ représente un groupe alkyle substitué ou non substitué, ou un groupe aryle substitué ou non substitué ; R₂ représente un substituant ; n représente un entier de 0 à 4 ; quand n est 2 ou plus, R₂s peuvent être identiques ou différents, et R₂s peuvent se liés les uns aux autres pour former un cycle ; R₃ représente un atome d'hydrogène ou un substituant ; et R₄ représente un groupe alkyle substitué ou non substitué.

2. Procédé selon la revendication 1, dans lequel la base est une base organique, un hydrure métallique, un alkoxyde métallique, un hydroxyde métallique, une base de l'acide carbonique ou une base de l'acide carboxylique.

3. Procédé selon la revendication 1, dans lequel, dans la réaction de cyclisation du composé représenté par la formule (II), le composé de la formule (II) et la base sont utilisés selon un rapport molaire de 1:0,001 à 1:100.

4. Procédé de production d'un composé de 1,2,4-benzothiadiazine-1,1-dioxyde représenté par la formule (I) selon la revendication 1, comprenant de faire réagir un composé de 2-aminobenzènesulfonamide représenté par la formule (III) avec un composé de 1,1-dialkoxyalkène représenté par la formule (IV) ou avec un composé orthoester représenté par la formule (V), en présence d'un acide, pour obtenir un composé iminoéther représenté par la formule (II) selon la revendication 1, et soumettre ensuite le composé iminoéther à une réaction de cyclisation, en présence d'une base : 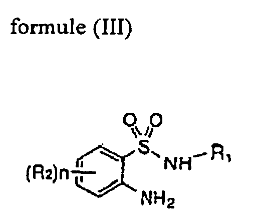 dans laquelle, dans les formules (I) à (III), R₁ à R₃ sont identiques à ceux de la revendication 1 ; 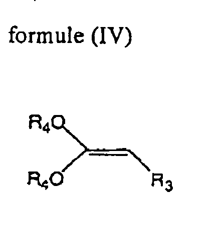 dans laquelle, dans la formule (IV), R₃ représente un atome d'hydrogène ou un substituant ; et R₄ représente un groupe alkyle substitué ou non substitué ; et 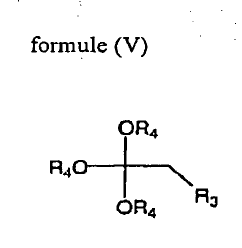 dans laquelle, dans la formule (V), R₃ représente un atome d'hydrogène ou un substituant ; et R₄ représente un groupe alkyle substitué ou non substitué.

5. Procédé selon la revendication 4, dans lequel R₁ est un groupe alkyle ayant de 1 à 60 atomes de carbone au total ou un groupe aryle ayant de 6 à 60 atomes de carbone au total.

6. Procédé selon la revendication 4, dans lequel R₃ est un atome d'hydrogène, un groupe alkyle, un groupe aryle, un groupe alkoxycarbonyle, un groupe aryloxycarbonyle ou un groupe carbamoyle.

7. Procédé selon la revendication 4, dans lequel l'acide est un acide inorganique, un acide carboxylique organique, un acide sulfonique organique ou un acide de Lewis.

8. Procédé selon la revendication 4, dans lequel la base est une base organique, un hydrure métallique, un alkoxyde métallique, un hydroxyde métallique, une base de l'acide carbonique ou une base de l'acide carboxylique.

9. Procédé selon la revendication 4, dans lequel, dans la réaction entre le composé représenté par la formule (III) et le composé représenté par la formule (IV) ou la formule (V), le composé représenté par la formule (III) et l'acide sont utilisés selon un rapport molaire de 1:0,001 à 1:100.

10. Procédé selon la revendication 4, dans lequel, dans la réaction de cyclisation du composé représenté par la formule (II), le composé de formule (II) et la base sont utilisés selon un rapport molaire de 1:0,001 à 1:100.

11. Procédé selon l'une quelconque des revendications 1 à 3, dans lequel R₁ dans les formules (I) et (II) représente un groupe alkyle substitué ou non substitué.

12. Procédé selon l'une quelconque des revendications 4 à 10, dans lequel R₁ dans les formules (I), (II) et (III) représente un groupe alkyle substitué ou non substitué.

13. Procédé selon l'une quelconque des revendications précédentes, dans lequel le solvant de réaction est le toluène ou le xylène.

14. Procédé selon la revendication 13, dans lequel le solvant de réaction est le toluène.
